Europäisches Patentamt

European Patent Office

Office européen des brevets

(11) Veröffentlichungsnummer: **0 452 543 B1**

## EUROPÄISCHE PATENTSCHRIFT

(45) Veröffentlichungstag der Patentschrift: **04.05.94**

(21) Anmeldenummer: **90122044.2**

(22) Anmeldetag: **17.11.90**

(51) Int. Cl.⁵: **B25D 9/14**, B25D 9/26, A61B 17/16

(54) Pneumatisches Schlagwerkzeug.

(30) Priorität: **20.04.90 CH 1345/90**

(43) Veröffentlichungstag der Anmeldung:
**23.10.91 Patentblatt 91/43**

(45) Bekanntmachung des Hinweises auf die
Patenterteilung:
**04.05.94 Patentblatt 94/18**

(84) Benannte Vertragsstaaten:
**AT BE CH DE DK ES FR GB GR IT LI LU NL SE**

(56) Entgegenhaltungen:
**WO-A-89/06516**
**CH-A- 661 239**
**DE-A- 3 148 708**
**FR-A- 2 339 751**

(73) Patentinhaber: **IMT INTEGRAL MEDIZINTECH-
NIK AG**
**Stationsstrasse 33**
**CH-6373 Ennetbürgen(CH)**

(72) Erfinder: **Mandanis, Georges**
**Hirtenhofstrasse 40 a**
**CH-6005 Luzern(CH)**

(74) Vertreter: **Blum, Rudolf Emil Ernst et al**
**c/o E. Blum & Co**
**Patentanwälte**
**Vorderberg 11**
**CH-8044 Zürich (CH)**

**Beschreibung**

Die Erfindung betrifft ein pneumatisches Schlagwerkzeug gemäss Oberbegriff des Patentanspruches 1.

Ein pneumatisches Schlagwerkzeug für Knochenbearbeitungswerkzeuge, insbesondere für Raspeln zur Bearbeitung der Lagerflächen von Knochen zur Aufnahme eines künstlichen Gelenkteiles, ist in der CH-A-661 239 beschrieben. Es hat sich indes gezeigt, dass die Wirkung eines solchen Werkzeuges trotz des lockernden Rückschlages auf das Bearbeitungswerkzeug nicht optimal ist, weil der rücktreibende Impuls zu schwach ist, um das Werkzeug jederzeit sicher aus der Knochenmarkhöhle zu entfernen.

Das Dokument WO-A-89 06 516 zeigt ferner ein Schlagwerkzeug, bei dem entweder ein Vorwärtsschlag oder ein Rückwärtsschlag ausgeführt wird, je nachdem, ob der Operateur am Griffteil des Schlagwerkzeuges zieht oder auf dieses Druck ausübt. Die Schlagkraft ist dabei stark variabel, je nach Grösse der ausgeübten Zug- oder Druckkraft.

Der Erfindung liegt deshalb die Aufgabe zugrunde, ein pneumatisches Schlagwerkzeug zu schaffen, das ein schnelles und möglichst patientenschonendes Herausarbeiten des Knochenmaterials bzw. Bearbeiten der Lagerfläche für den künstlichen Gelenkteil ermöglicht. Auch bei einem Schlagwerkzeug für allgemeine Bearbeitungszwecke (z.B. im Bauwesen), kann es vorteilhaft sein, einen wirksamen Rückschlag auf das Werkzeug zu erzeugen.

Dies wird bei einem pneumatischen Schlagwerkzeug der eingangs genannten Art mit den Merkmalen des kennzeichnenden Teiles des Patentanspruches 1 erreicht.

Bei einer bevorzugten Ausführungsart ist im Innern des Zylinders ein Kraftaufnahmeelement angeordnet. Durch dieses kann der Stossimpuls des Kolbens direkt auf das Werkzeug geleitet werden. Dies ermöglicht eine leichte und kostengünstige Ausführung des Zylinders, z.B. aus Kunststoff oder Aluminium.

Dabei sind verschiedene Ausführungsformen möglich. So kann der vortreibende Schlag durch den Aufprall des Kolbens beim Kolbenvorlauf auf den Zylinderdeckel erfolgen und der rücktreibende Schlag durch den Aufprall des Kolbens am Kraftaufnahmeelement. Umgekehrt kann der vortreibende Schlag am Kraftaufnahmeelement erfolgen und der rücktreibende Schlag durch den Aufprall des Kolbens am hinteren, werkzeugabgewandten Zylinderdeckel. Bevorzugterweise erfolgt aber sowohl der vortreibende als auch der rücktreibende Schlag durch das Aufprallen des vorlaufenden bzw. rücklaufenden Kolbens am Kraftaufnahmeelement. In diesem Fall können auch die Zylinderdeckel aus Kunststoff oder Aluminium bestehen.

Bei einer weiteren bevorzugten Ausführungsart ist zudem die Lage des Kraftaufnahmeelementes im Zylinder verstellbar. Dadurch sind verschiedene Betriebszustände möglich. So kann je nach Einstellung mit dem selben Schlagwerkzeug wahlweise nur ein vortreibender Schlag oder nur ein rücktreibender Schlag erzeugt oder eine Leerlaufstellung gewählt werden.

Im folgenden sollen Ausführungsbeispiele näher erläutert werden. Dabei zeigt:

Fig. 1 eine teilweise geschnittene Ansicht eines Ausführungsbeispieles des Schlagwerkzeuges;

Fig. 2a bis 2d verschiedene Arbeitsstellungen des Kolbens bei einem Schlagwerkzeug gemäss Fig. 1;

Fig. 3 eine teilweise geschnittene Ausführungsart eines weiteren Schlagwerkzeuges;

Fig. 4a bis 4e verschiedene Arbeitsstellungen einer weiteren Ausführungsart bei Vorwärtsschlag;

Fig. 5a bis 5d dieselbe Ausführung bei Rückwärtsschlag, und

Fig. 6a bis 6d dieselbe Ausführung bei Leerlauf;

Fig. 7 ein Beispiel für die Anordnung des Kraftaufnahmeelementes am Zylinder.

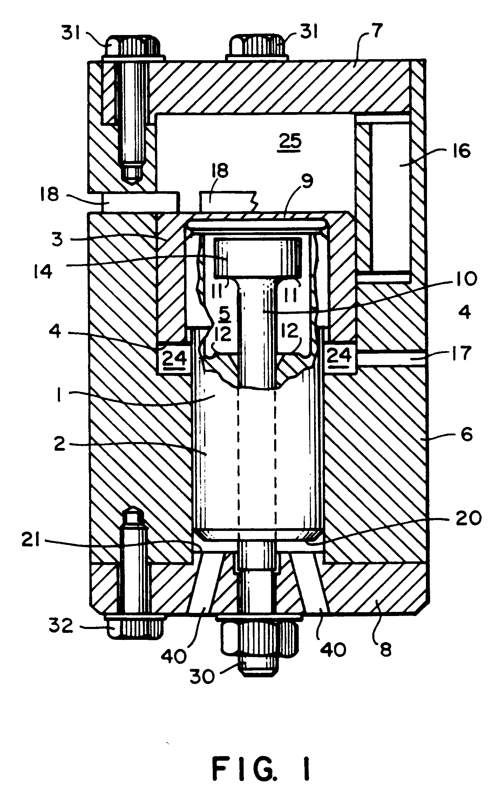

Fig. 1 zeigt ein erstes Ausführungsbeispiel des pneumatischen Schlagwerkzeuges.

Dabei ist ein Kolben 1 in einem Zylinder 6, der mit einem oberen Deckel 7 und einem unteren, mit Auslässen 40 versehenen, Deckel 8 verschlossen ist, verschieblich angeordnet. Die Deckel 7,8 sind mit Gewindebolzen 31 bzw. 33 am Zylinder 6 befestigt. Der Kolben 1 ist ein Stufenkolben mit einem oberen Kolbenteil 3 mit grösserem Durchmesser als der untere Kolbenteil 2. Die von der Stufe des Kolbens gebildete Ringfläche ist mit 4 bezeichnet. Entsprechend der Stufung des Kolbens 1 ist auch der Zylinder 6 mit zwei verschiedenen Innendurchmessern versehen. Am unteren Deckel 8 ist eine Säule 10 als Kraftaufnahmeelement befestigt, welche in den Zylinder und den Kolben hineinragt. An ihrem aus dem Zylinder hinausragenden Ende 30 wird eine (nicht gezeigte) Fassung für das Werkzeug, insbesondere eine Knochenraspel, befestigt. Das im Zylinder bzw. im Kolben 1 befindliche Ende der Säule 10 weist einen aufgeweiteten Kopf 14 auf, der mit mindestens einer ersten Aufprallfläche 11 für den Kolben versehen ist. Entsprechend ist der Kolben 1 mit einer Bohrung für den Säulenschaft und mit einem Hohlraum 5 grösseren Durchmessers als die Bohrung versehen, in welchem der Säulenkopf 14 aufgenommen ist. Am Uebergang von der Bohrung zum Hohlraum ist am Kolben kolbeninnenseitig eine Schlagfläche 12 ausgebildet, welche - wie später beschrieben wird - die Aufprallfläche 11 beaufschlagt.

Bei dem gezeigten Ausführungsbeispiel ist am unteren Zylinderdeckel 8 zylinderinnenseitig eine zweite Aufprallfläche 21 vorgesehen, bzw. am unteren Kolbenende kolbenaussenseitig eine zweite Schlagfläche 20 vorgesehen.

Der obere Kolbenteil 3 unterteilt den Zylinderbereich mit grössserem Durchmesser in zwei Zylinderräume 24 bzw. 25, welche jeweils von den Kolbenflächen 4 bzw. 9 begrenzt werden. Die Fläche 4 ist ringförmig und flächenmässig kleiner als die Fläche 9. Die Zylinderräume 24 bzw. 25 besitzen ein - je nach Stellung des Kolbens im Zylinder - variables Volumen. Die beiden Räume 24, 25 sind durch mindestens einen Ueberströmkanal 16 in der Zylinderwandung verbunden, dessen Einlass durch den Kolbenteil 3 gesteuert wird, d.h. die Lage des Kolbens bestimmt, ob die Zylinderräume 24, 25 in Verbindung stehen oder nicht. In den unteren Zylinderraum 24 mündet ferner ein Drucklufteinlass 17 für die Zufuhr von Druckluft in den Zylinder aus einer nicht dargestellten Druckluftquelle. Im oberen Zylinderraum 25 ist mindestens eine Auslassöffnung 18 vorgesehen, welche ebenfalls durch den Kolbenteil 3 geöffnet oder geschlossen wird.

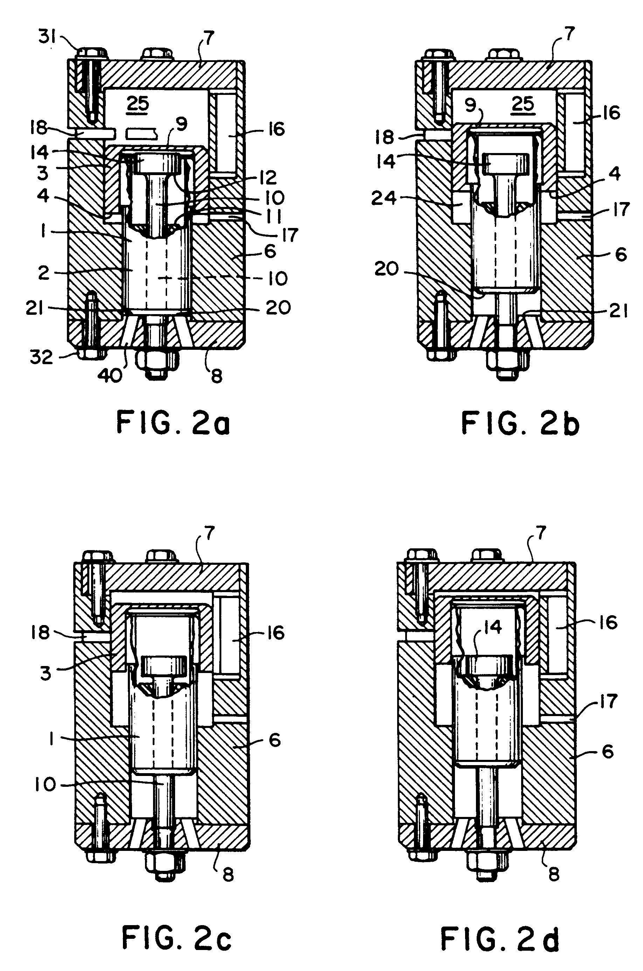

Anhand der Fig. 2a bis 2d soll nun die Funktion des Schlagwerkzeuges erläutert werden. Fig. 2a zeigt dabei den Kolben 1 an seinem unteren Totpunkt, bei welchem der Kolben 1 mit seiner Schlagfläche 20 auf den Zylinderdeckel 8 bzw. die Aufprallfläche 21 auftrifft und der - nicht gezeigten Knochenraspel - einen vortreibenden Stossimpuls verleiht. Durch den Drucklufteinlass 17 strömt Druckluft mit vorzugsweise ca. 7 bar Druck in den unteren Zylinderraum 24 und wirkt über die ringförmige Fläche 4 auf den Kolben 1 ein. Da im oberen Zylinderraum 25 der Auslass 18 geöffnet ist, wirkt dort nur der Umgebungsdruck von ca. 1 bar. Die Druckdifferenz zwischen den Zylinderräumen bewirkt eine Beschleunigung des Kolbens nach oben. Bei der Verschiebung des Kolbens 1 werden die Auslassöffnungen 18 durch den Kolben geschlossen (Fig. 2b). Es erfolgt nun eine Kompression der Luft im oberen, kleiner werdenden Zylinderraum 25. In Fig. 2c ist der Kolben 1 in der Stellung gezeigt, in welcher die Oeffnung des Ueberströmkanals 16 erfolgt. Durch den Kanal 16 wird auch der obere Zylinderraum 25 mit der Druckluftquelle verbunden, und es herrscht nun auch im Raum 25 ein Druck von ca. 7 bar. Der Kolben bewegt sich aufgrund seiner Bewegungsenergie weiterhin nach oben, wird aber gebremst, da nun auf die obere Kolbenfläche 9, welche grösser ist als die ringförmige Kolbenfläche 4, ebenfalls der Druck von 7 bar wirkt, auf den Kolben somit eine nach unten gerichtete Kraft einwirkt. Fig. 2d zeigt, wie der Kolben 1 seinen oberen Totpunkt erreicht, wobei er bei seiner gebremsten Aufwärtsbewegung mit seiner Restgeschwindigkeit am Kraftaufnahmeelement 10 aufprallt. Der Aufprall erfolgt dabei mittels der Schlagfläche 12 des Kolbens an der Aufprallfläche 11 des Säulenkopfes 14. Der Aufprall wirkt sich als rücktreibende Kraft am Werkzeug aus. Nach dem Aufprall erfolgt eine Beschleunigung des Kolbens nach unten infolge des Druckes von 7 bar auf die Fläche 9. Bei der Abwärtsbewegung des Kolbens wird der Kanal 16 geschlossen, und es erfolgt Expansion im oberen Zylinderraum und nachfolgend ein Ausströmen der Luft durch den sich öffnenden Auslass 18. Der Kolben bewegt sich, aufgrund seiner Bewegungsenergie, gegen die Bremskraft, bewirkt von der druckluftbeaufschlagten Fläche 4, weiter nach unten und erreicht erneut den unteren Totpunkt beim Aufprall mit Restgeschwindigkeit auf den Zylinderdeckel 8 (Figur 2a).

Damit das Verhältnis von vorwärts gerichtetem zu rückwärts gerichtetem Stossimpuls auf das Werkzeug ca. 1 beträgt, erfolgt die Dimensionierung der Vorrichtung unter Einhaltung der folgenden Regeln: Ein erster Parameter A ist definiert wie folgt:

$$ A \;=\; \frac{A_1}{A_2}\ \frac{P_O}{P_2} $$

wobei

$A_1$ = Grösse der oberen Fläche 9 des Kolbens
$A_2$ = Grösse der ringförmigen Fläche 4 des Kolbens
$P_0$ = Wert des Umgebungsdrucks (ca. 1 bar)
$P_2$ = Wert des Drucks der Druckluftquelle (ca. 7 bar)

Zwei weitere Parameter sind definiert wie folgt:

$$ V_u \;=\; \frac{V_{12}}{V_A} \quad \text{und} \quad V_O \;=\; \frac{V_T}{V_A}\ ; $$

3

wobei $V_A$ das Volumen des Ueberströmkanals 16 plus das Volumen des oberen Zylinderraumes 25 bei der Kolbenstellung ist, bei welcher der Auslass 18 vom Kolben gerade geschlossen wird (Figur 2b);

$V_{12}$ das Volumen des Ueberströmkanals 16 plus des oberen Zylinderraums 25 bei der Kolbenstellung ist, bei welcher der Ueberströmkanal 16 gerade geöffnet wird (Figur 2c) und

$V_T$ das Volumen des Ueberströmkanals 16 plus des oberen Zylinderraums 25 im oberen Kolbentotpunkt ist (Figur 2d).

Das gewünschte Stossimpulsverhältnis von 1 wird erreicht, wenn die Parameter im wesentlichen in folgenden Bereichen liegen:

$$O,1 \leq A \leq O,5$$
$$O \leq V_o \leq V_u \leq 1$$

Vorzugsweise liegen indes die Parameter $V_o$, $V_u$ im Bereich von 0,1 bis 0,8.

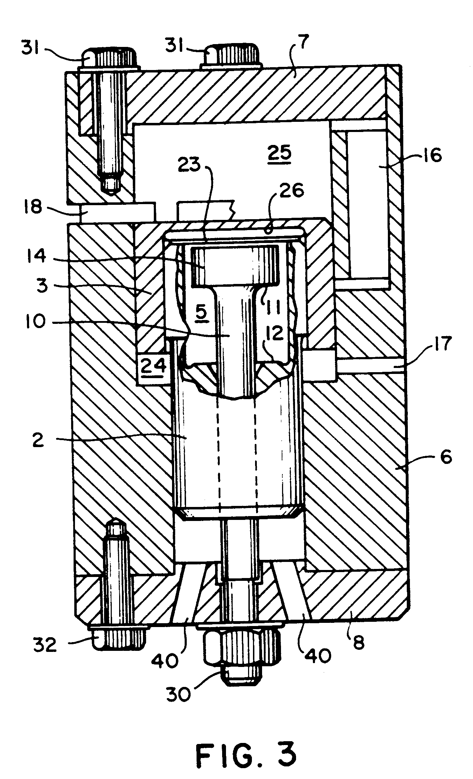

Figur 3 zeigt eine weitere Ausführungsart, bei welcher gleiche Teile wie bei der vorstehend beschriebenen Ausführung mit gleichen Bezugsziffern bezeichnet sind. Der Unterschied zur vorherigen Ausführung liegt darin, dass durch Verkürzung des unteren Kolbenteils 2, der Aufprall des Kolbens beim Kolbenvorlauf nicht auf den Zylinderdeckel 8 erfolgt, sondern, wie beim Rücklauf, auf den Kopf 14 des Kraftaufnahmeelementes. Zu diesem Zweck ist der Kopf 14 mit der zweiten Aufprallfläche 23 versehen, und die Innenfläche des Kolbenbodens bildet die zweite Schlagfläche 26. Der Vorteil dieser Ausführung liegt darin, dass die Schlagkräfte kräfte nur auf das Kraftaufnahmeelement 10 einwirken, welches mit dem Werkzeug (der Raspel) in Verbindung steht. Der Zylinder, inklusive der Zylinderdeckel 7 und 8, muss keine Schlagkräfte aufnehmen und kann daher aus billigeren und leichteren Materialen gefertigt werden, z.B. aus Aluminium oder Kunststoff.

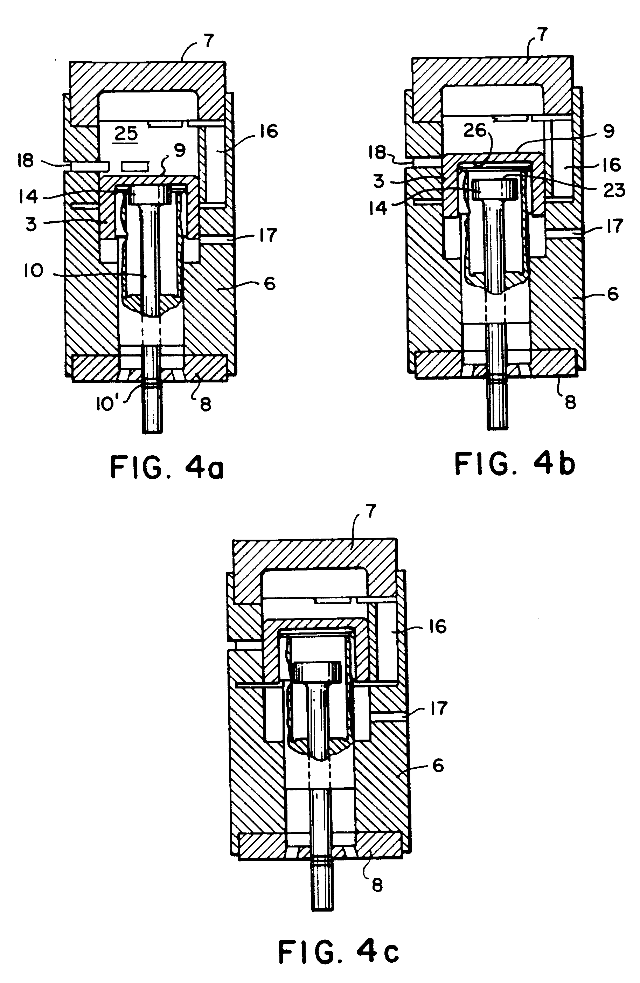

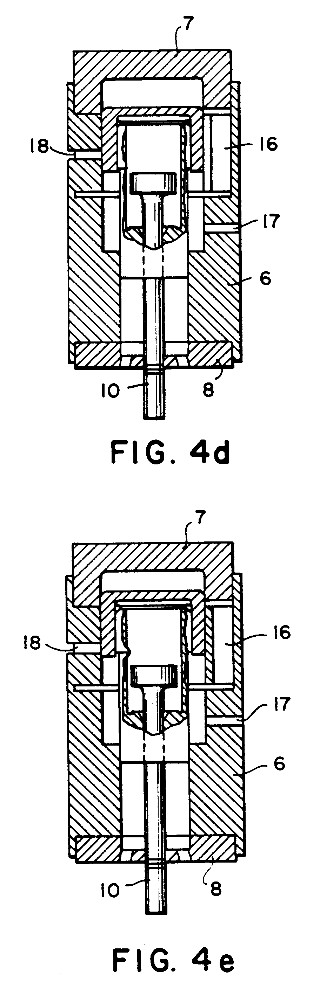

Figuren 4a bis 4e, 5a bis 5d und 6a bis 6d zeigen verschiedene Betriebsarten einer weiteren Ausführungsform der Erfindung. Bei dieser Ausführungsform ist das Schlagwerkzeug derart einstellbar, dass Schläge wahlweise nur vorwärts gerichtet, nur rückwärts gerichtet oder gar nicht erfolgen (Leerlaufbetrieb). Diese Einstellmöglichkeit wird erreicht, indem das Kraftaufnahmeelement 10 in verschiedenen Stellungen entlang der Zylinderlängsachse fixierbar ist. Figuren 4a bis 4e zeigen den Betrieb des Schlagwerkzeuges für die Ausführung eines vorwärts gerichteten, die Raspel im Knochen vortreibenden Schlages. Gleiche Bezugszeichen in den Figuren bezeichnen dabei gleiche Teile wie bei den vorstehend beschriebenen Ausführungsformen. An der Säule 10 ist eine Markierung 10' dargestellt, um die Verschiebung der Säule 10 bei den verschiedenen Stellungen in den Figuren 4, 5 und 6 besser kenntlich zu machen.

In Figur 4a ist der Kolben 1 an seinem unteren Totpunkt gezeigt, bei welchem die Schlagfläche 26 des Kolbens auf die Aufprallfläche 23 des Kopfes 14 auftrifft und auf das Werkzeug einen vorwärts treibenden Schlag ausübt. Durch den Druck von 7 bar auf die Ringfläche 4 wird der Kolben 1 vom unteren Totpunkt aus in Richtung auf den Zylinderdeckel 7 angetrieben. Der Kolben schliesst dabei den Auslass 18 (Figur 4b) und im Zylinderraum 25 erfolgt Kompression. Bei der Kolbenstellung gemäss Figur 4c wird der Ueberströmkanal 16 geöffnet, und damit die Druckluftzufuhr in den oberen Zylinderraum 25 frei gegeben. Es erfolgt eine Bremsung des steigenden Kolbens 1 durch den auf die Fläche 9 ausgeübten Druck.

Figur 4d zeigt das Schliessen des Ueberströmkanals durch die obere Kolbenkante bei dieser Ausführungsform. Im Deckel 7 ist ein Luftvolumen als Bremsvolumen eingeschlossen, welches dem weiter steigenden Kolben eine stark zunehmende Bremskraft entgegensetzt. Figur 4e zeigt den oberen Totpunkt des Kolbens, welcher durch die sanfte Bremsung des Kolbens mittels dieses Bremsvolumens erreicht wird, ohne dass der Kolben mit seiner Schlagfläche 12 auf der Aufprallfläche 11 auftrifft. Die Rückwärtsbewegung des Kolbens wird somit ohne Schlag auf das Werkzeug beendet. Durch die Expansion des Bremsvolumens erfolgt eine erneute Beschleunigung des Kolbens nach unten, wobei der Ueberströmkanal 16 durch die obere Kolbenkante wieder geöffnet wird, und der Druck von 7 bar auf die Fläche 9 wirkt, was den Kolben weiter beschleunigt. Nach dem Schliessen des Ueberströmkanals 16 durch die untere Kolbenkante erfolgt Expansion im oberen Kolbenraum 25 und nachfolgend wird der Auslass 18 geöffnet. Eine Bremsung des Kolbens 1 erfolgt zwar durch den Druck auf die Ringfläche 4, der untere Kolbentotpunkt ergibt sich aber durch den Aufprall des Kolbens 1 auf die Fläche 23 (Figur 4a). Es wird somit jeweils nur ein Schlag beim unteren Totpunkt erzeugt, während im oberen Totpunkt eine sanfte, schlagfreie Bremsung erfolgt.

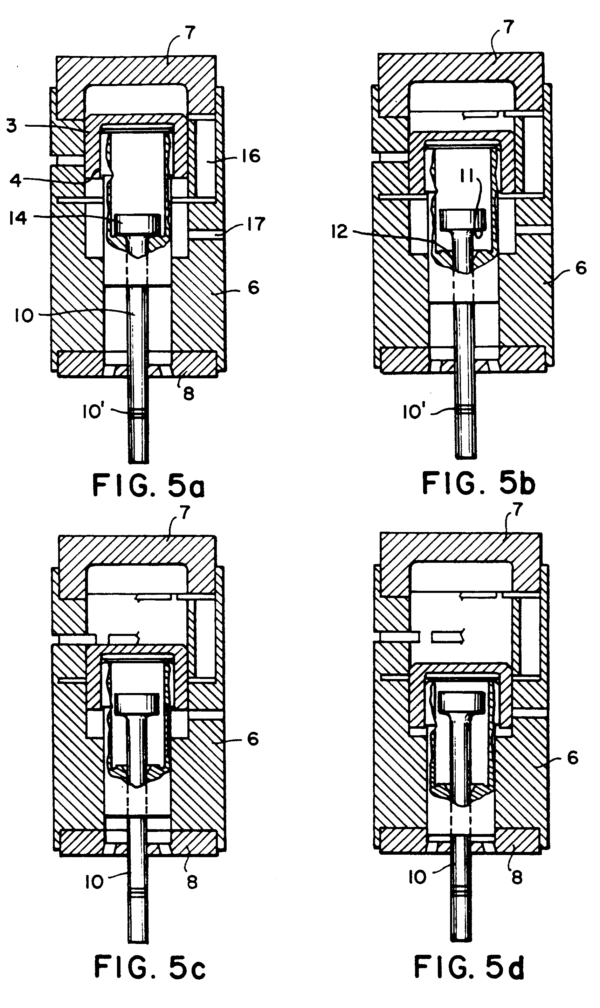

Figuren 5a bis 5d zeigen den umgekehrten Betrieb: Schlag beim oberen Kolbentotpunkt und damit das Rücktreiben des Raspels, sanftes, schlagloses Bremsen des Kolbens im unteren Kolbentotpunkt. Für diese Betriebsart wird die Säule 10 entlang der Zylinderlängsachse relativ zum Zylinder verschoben, was im Vergleich mit Figur 4 an der Lage der Markierung 10' in Bezug auf den Zylinderdeckel 8 ersichtlich ist.

Figur 5a zeigt den Kolben im oberen Totpunkt, bei welchem der Schlag durch den Aufprall des Kolbens 1 an der Aufprallfläche 11 des Kopfes 14 erfolgt. Der Kolben 1 wird anschliessend nach unten in Richtung des Deckels 8 beschleunigt, wobei 7 bar Druck auf die Fläche 9 und die kleinere, ringförmige Fläche 4 wirken (Figur 5b).

Figur 5c zeigt die Bremsphase des Kolbens, bei welcher 7 bar Druck auf die Ringfläche 4 wirken und 1 bar Druck, bei offenem Auslass 18, auf die Fläche 9. Durch das Bremsvolumen im Raum 24 bei geschlossenem Einlass 17, erfolgt bei dieser Ausführung eine sanfte Bremsung im unteren Totpunkt (Figur 5d), ohne dass der Kolben auf der Aufprallfläche 23 aufprallt und einen Schlag bewirkt, da die Säule 10 gegenüber den Figuren 4 verschoben angeordnet ist. Die Expansion des Bremsvolumens treibt den Kolben erneut nach oben, wobei der Drucklufteinlass 17 geöffnet und dadurch der Kolben nach oben beschleunigt wird.

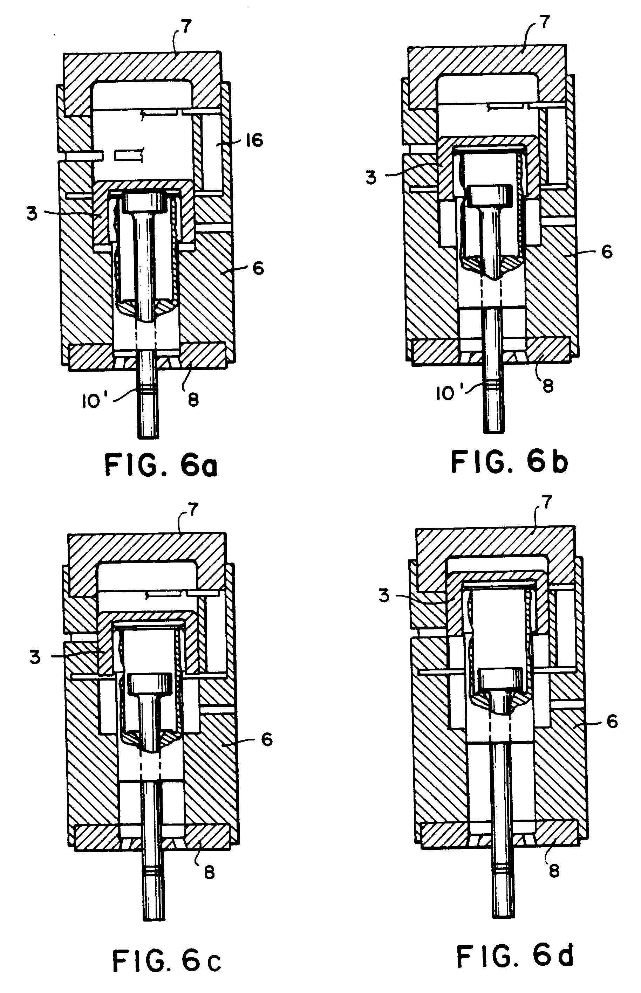

Figuren 6a bis 6d zeigen die Leerlaufstellung. Die Säule 10 ist dabei gegenüber dem Zylinder in eine Lage gebracht, welche zwischen den Stellungen von Figur 4 und Figur 5 liegt. In dieser Stellung erfolgt sowohl beim oberen Totpunkt wie beim unteren Totpunkt eine sanfte Bremsung mittels des jeweiligen Bremsvolumens, d.h. es erfolgt in beiden Endlagen des Kolbens kein Aufprall auf der Säule 10. Das Schlagwerkzeug ist zwar in Funktion, erzeugt aber keinen Schlag auf das Bearbeitungswerkzeug. Figur 6a zeigt dabei den gebremsten Kolben im unteren Totpunkt ohne Kontakt mit der Aufprallfläche 23, Figur 6b die durch Druckausübung auf die Ringfläche 4 beschleunigte Aufwärtsbewegung des Kolbens, Figur 6c das Oeffnen des Ueberströmkanals 16 und Figur 6d den durch das Bremsvolumen im Deckel 7 gebremsten Kolben 1 im oberen Totpunkt ohne Kontakt mit der Aufprallfläche 11. Die Verstellung der Säule 10 relativ zum Zylinder 6 kann z.B. dadurch erfolgen, dass die Säule 10 im Bereich des Deckels 8 mit einem Gewinde versehen ist und die Bohrung für die Säule im Deckel 8 ein entsprechendes Gewinde aufweist. Durch Drehen der Säule ergibt sich so eine Verschiebung derselben in der Zylinderlängsache. Durch optische Markierungen an der Säule können die drei Betriebsstellungen gekennzeichnet werden.

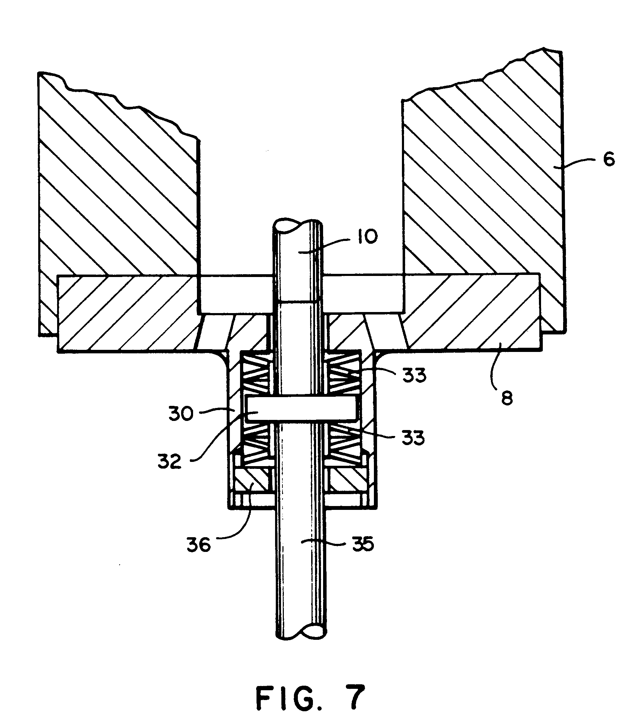

Figur 7 zeigt indes eine bevorzugte Anordnung des Kraftaufnahmeelementes 10 am Zylinder 6 bzw. am Deckel 8. Der Deckel 8 weist dazu eine Hülse 30 auf, in welchem ein Ring 32 mit Innengewinde zwischen Tellerfedern 33 gehalten wird. Am Innengewinde des Rings 32 greift das Aussengewinde 35 der Säule 10. Die Hülse 30 ist durch einen Deckel 36 verschlossen, welcher die Tellerfedern in der Hülse hält. Bei diesem Beispiel erfolgt die Verstellung der Säule 10 ebenfalls durch Drehung derselben, wobei das Gewinde 35 bzw. das Innengewinde des Rings 32 vorzugsweise eine grosse Steigung aufweist. Die den Ring 32 haltenden Tellerfedern sorgen dafür, dass die Schläge auf die Säule 10 nur gedämpft auf den Deckel 8 und den Zylinder 6 übertragen werden.

**Patentansprüche**

**1.** Pneumatisches Schlagwerkzeug mit einem Zylinder (6), einer Halterung für ein Bearbeitungswerkzeug und mit einem, im Zylinder verschieblich angeordneten, als Schlagelement wirkenden Kolben (1) mit einer, für den auf das Werkzeug hin gerichteten Kolbenvorlauf mit Druckluft beaufschlagbaren ersten Fläche (9), einer der ersten Fläche (9) entgegengerichteten, druckluftbeaufschlagbaren zweiten Fläche (4) für den Kolbenrücklauf, sowie mit zwei Schlagflächen (12, 20; 12, 26), durch welche an den beiden Totpunkten des Kolbens jeweils ein, das Werkzeug vortreibender, bzw. ein entgegengesetzter, das Werkzeug lockernder, Stoss auf das Werkzeug bewirkbar ist, und wobei eine Druckluftzufuhr (17) und mindestens ein Auslass (18) für die Druckluft vorgesehen sind, dadurch gekennzeichnet, dass mindestens ein vom Kolben gesteuerter Ueberströmkanal (16) zwischen der ersten und der zweiten Fläche (9, 4) vorgesehen ist, und dass der Kolben (1) den Zylinder in mindestens zwei Zylinderräume (24, 25) unterteilt, wobei der erste Zylinderraum (25) von der ersten Fläche (9) mit der Grösse $A_1$ begrenzt ist, und der zweite Zylinderraum (24) von der zweiten Fläche (4) mit der Grösse $A_2$ begrenzt ist, und wobei der Ueberströmkanal (16) in der Zylinderwandung zwischen dem ersten und zweiten Zylinderraum, der Auslass (18) als eine vom Kolben gesteuerte Verbindung des ersten Zylinderraumes (25) mit dem Umgebungsdruck $p_o$ und eine in den zweiten Zylinderraum (24) mündende Druckluftquelle mit dem Druck $p_2$ vorgesehen sind, und dass ein erster Parameter A definiert als

$$A \quad = \quad \frac{A_1}{A_2} \quad \cdot \quad \frac{p_o}{p_2}$$

und dass $V_A$ das Volumen des ersten Zylinderraumes (25) und des Ueberströmkanals (16) in dem Zeitpunkt ist, da der rücklaufende Kolben den Auslass (18) schliesst, $V_{12}$ das Volumen des ersten Zylinderraumes (25) und des Ueberströmkanals (16) in dem Zeitpunkt ist, da der rücklaufende Kolben den Ueberströmkanal (16) freigibt, und $V_T$ das Volumen des ersten Zylinderraumes und des Ueberströmkanals im Totpunkt des Kolbenrücklaufs ist, und dass zwei weitere Parameter $V_u$ und $V_o$ definiert sind als

$$V_u \;=\; \frac{V_{12}}{V_A} \quad ; \quad V_o \;=\; \frac{V_T}{V_A}$$

und dass die Parameter innerhalb folgender Bereiche liegen

$0{,}1 \leq A \leq 0{,}5$

und

$0{,}1 \leq V_o \leq V_u \leq 0{,}8,$

um bei dem vorbestimmten Druck $p_2$ einen im wesentlichen jeweils gleich grossen Stossimpuls beim Kolbenvorlauf und beim Kolbenrücklauf zu erzielen.

**2.** Pneumatisches Schlagwerkzeug nach Anspruch 1, gekennzeichnet durch ein am werkzeugseitigen Zylinderabschluss (8) in den Zylinderinnenraum ragendes, im wesentlichen koaxial zur Werkzeuglängsachse verlaufendes Kraftaufnahmeelement (10), welches vom hohl ausgeführten Kolben (1) umschlossen wird und an seinem vom Werkzeug entfernten Ende (14) eine Aufprallfläche (11) trägt, die beim Totpunkt des Kolbenrücklaufs von der im Kolbenhohlraum (5) ausgebildeten Schlagfläche (12) beaufschlagbar ist.

**3.** Pneumatisches Schlagwerkzeug nach Anspruch 2, dadurch gekennzeichnet, dass das Kraftaufnahmeelement (10) eine zweite Aufprallfläche (23) trägt, welche beim Totpunkt des Kolbenvorlaufs von der anderen, vom kolbeninnenseitigen Kolbenboden gebildeten Schlagfläche (26) beaufschlagbar ist.

**4.** Pneumatisches Schlagwerkzeug nach Anspruch 3, dadurch gekennzeichnet, dass das Kraftaufnahmeelement (10) in der Zylinderlängsachse verschieblich einstellbar ist, wobei in einer ersten Einstellage jeweils die zweite Aufprallfläche (23) beim Totpunkt des Kolbenvorlaufs beaufschlagbar ist, und der Kolben beim Totpunkt des Kolbenrücklaufs durch ein Druckluftpolster abbremsbar ist, in einer zweiten Einstellage jeweils die erste Aufprallfläche (11) beim Totpunkt des Kolbenrücklaufs beaufschlagbar ist, und der Kolben beim Totpunkt des Kolbenvorlaufs durch ein Druckluftpolster abbremsbar ist, und in einer dritten Einstellage keine der beiden Aufprallflächen (11, 23) beaufschlagbar ist, und der Kolben bei beiden Totpunkten durch jeweils ein Druckluftpolster abbremsbar ist.

**5.** Pneumatisches Schlagwerkzeug nach Anspruch 4, dadurch gekennzeichnet, dass das Kraftaufnahmeelement (10) an einem im Deckel (8) federnd gehaltenen Element (32) verschieblich angeordnet ist.

**6.** Pneumatisches Schlagwerkzeug nach einem der Ansprüche 2 bis 4, dadurch gekennzeichnet, dass der Kolben (1, 2, 3) und das Kraftaufnahmeelement (10) aus Metall bestehen und der Zylinder (6, 7, 8) aus Aluminium oder faserverstärktem Kunststoff gebildet ist.

## Claims

**1.** Pneumatic percussion tool with a cylinder (6), a support for a working tool and a piston (1) arranged for displacement in the cylinder and acting as striking member, and with a first surface (9) to be acted upon by compressed air for the forward movement of the piston directed to the tool, a second surface (4) opposite of the first surface (9) and to be acted upon by compressed air for the backwards motion of the piston, and with two striking surfaces (12, 20; 12, 26), by means of which at both dead centers of the piston a respective working tool advancing and opposite, resp., the working tool loosening strike onto the working tool can be effected, and whereby a pressurized air supply (17) and at least one outlet

6

(18) for the pressurized air are foreseen, characterized in that at least one transfer channel (16) controlled by the piston is foreseen between the first and second surfaces (9, 4), and that the piston (1) divides the cylinder into at least two cylinder chambers (24, 25), whereby the first cylinder chamber (25) is defined by the first surface (9) with the size $A_1$ and the second cylinder chamber (24) is defined by the second surface (4) with the size $A_2$, and whereby the transfer channel (16) is foreseen in the cylinder wall between the first and the second cylinder chamber, the outlet (18) as a connection between the first cylinder chamber (25) with the ambient pressure $p_o$ controlled by the piston, and a source of pressurized air with a pressure $p_2$ opening into the second cylinder chamber (24), and that a first parameter A is defined as

$$A = \frac{A_1}{A_2} \cdot \frac{p_o}{p_2}$$

and that $V_A$ is the volume of the first cylinder chamber (25) and of the transfer channel (16) at the moment when the piston moving backwards closes the outlet (18) off, $V_{12}$ is the volume of the first cylinder chamber (25) and of the transfer channel (16) at the moment when the piston moving backwards opens the transfer channel (16), and $V_T$ is the volume of the first cylinder chamber and the transfer channel at the dead center of the backwards movement of the piston, and in that two further parameters $V_u$ and $V_o$ are defined as

$$V_u = \frac{V_{12}}{V_A} \quad ; \quad V_o = \frac{V_T}{V_A}$$

and that the parameters are located within the following ranges

$0.1 \leqq A \leqq 0.5$

and

$0.1 \leqq V_o \leqq V_u \leqq 0.8,$

in order to obtain at the predetermined pressure $p_2$ a striking impulse at the forwards movement of the piston and at the backwards movement of a substantially same magnitude.

2. Pneumatic percussion tool according to claim 1, characterized by a force receiving element (10) projecting at the cylinder cover (8) at the side of the tool into the inner space of the cylinder and extending substantially coaxially to the longitudinal axis of the tool, which is encased by the hollow piston (1) and carries an impact surface (11) at its end remote from the tool which can be acted upon by the striking surface (12) formed in the hollow space (5) of the piston at the dead point of the backwards movement of the piston.

3. Pneumatic percussion tool according to claim 2, characterized in that the force receiving member (10) carries a second impact surface (23) which can be acted upon by the other striking surface (26) formed at the piston-head at the inner side of the piston at the dead center of the forwards movement of the piston.

4. Pneumatic percussion tool according to claim 3, characterized in that the force receiving element (10) is adjustable for displacement along the longitudinal axis of the cylinder, whereby in a respective first adjusted position the second impact surface (23) can be acted upon at the dead point of the forwards movement of the piston, and the piston can be braked at the dead point of the backwards movement by a pressurized air cushion, that in a respective second adjusted position the first impact surface (11) can be acted upon at the dead point of the backwards movement of the piston, and the piston can be braked by a pressurized air cushion at the dead point of the forward movement of the piston, and that

7

in a third adjusted position none of the two impact surfaces (11, 23) can be acted upon and the piston can be braked by a respective pressurized air cushion at both dead points.

5. Pneumatic percussion tool according to claim 4, characterized in that the force receiving member (10) is arranged for displacement at a member (32) held flexibly in the cover (8).

6. Pneumatic percussion tool according to one of the claims 2 to 4, characterized in that the piston (1, 2, 3) and the force receiving element (10) consist of metal and the cylinder (6, 7, 8) is formed of aluminium or fiber reinforced plastic material.

**Revendications**

1. Outil à percussion pneumatique comportant un cylindre (6), un support pour un outil d'usinage et un piston (1) agencé de façon coulissante dans le cylindre, agissant comme élément de percussion et comportant une première face (9), qui peut être sollicitée par de l'air comprimé pour la course d'avance du piston dirigée vers l'outil, une seconde face (4) pour la course de retour du piston, qui peut être sollicitée par de l'air comprimé et qui est orientée à l'opposé de la première face (9), et aussi deux faces de percussion (12, 20; 12, 26) par lesquelles respectivement un impact qui entraîne l'outil vers l'avant et respectivement un impact opposé qui dégage l'outil peuvent être effectués aux deux points morts du piston, et dans lequel sont prévus pour l'air comprimé une alimentation en air comprimé (17) et au moins un échappement (18), caractérisé en ce qu'il est prévu entre les première et seconde faces (9, 4) au moins un canal de passage (16) qui est commandé par le piston, et en ce que le piston (1) divise le cylindre en au moins deux chambres de cylindre (24, 25), la première chambre de cylindre (25) étant délimitée par la première face (9) de la dimension $A_1$ et la seconde chambre de cylindre (24) étant délimitée par la seconde face (4) de la dimension $A_2$, le canal de passage (16) étant prévu dans la paroi de cylindre entre les première et seconde chambres de cylindre, l'échappement (18) étant prévu sous la forme d'un raccordement, commandé par le piston, de la première chambre de cylindre (25) et de la pression d'environnement $p_0$, une source d'air comprimé à la pression $p_2$ et débouchant dans la seconde chambre de cylindre (24) étant prévue, et en ce qu'un premier paramètre A est défini par

$$A = \frac{A_1}{A_2} \cdot \frac{p_0}{p_2}$$

et en ce que $V_A$ est le volume de la première chambre de cylindre (25) et du canal de passage (16) au moment où le piston qui revient ferme l'échappement (18), $V_{12}$ est le volume de la première chambre de cylindre (25) et du canal de passage (16) au moment où le piston qui revient libère le canal de passage (16) et $V_T$ est le volume de la première chambre de cylindre et du canal de passage au point mort de la course de retour du piston, et en ce que deux autres paramètres $V_u$ et $V_o$ sont déterminés par

$$V_u = \frac{V_{12}}{V_A} \quad ; \quad V_o = \frac{V_T}{V_A}$$

et en ce que les paramètres sont compris dans les plages suivantes :

$0,1 \leqq A \leqq 0,5$

et

$0,1 \leqq V_o \leqq V_u \leqq 0,8,$

afin d'obtenir pour la pression $p_2$ prédéterminée une impulsion de choc chaque fois sensiblement de même grandeur pour la course en avant du piston et pour la course de retour du piston.

**2.** Outil à percussion pneumatique suivant la revendication 1, caractérisé par un élément de reprise de force (10) qui fait saillie par la fermeture du cylindre (8), du côté de l'outil, dans la chambre interne du cylindre, qui s'étend sensiblement coaxialement par rapport à l'axe longitudinal de l'outil, qui est entouré par le piston (1) réalisé de façon creuse et qui porte sur son extrémité (14) éloignée de l'outil une face d'impact (11) pouvant être sollicitée au point mort de la course de retour du piston, par la face de percussion (12) réalisée dans la cavité de piston (5).

**3.** Outil à percussion pneumatique suivant la revendication 2, caractérisé en ce que l'élément de reprise de force (10) comporte une seconde face d'impact (23) qui peut être sollicitée, au point mort de la course d'avance du piston, par l'autre face à percussion (26) formée par le fond du piston du côté interne du piston.

**4.** Outil à percussion pneumatique suivant la revendication 3, caractérisé en ce que l'élément de reprise de force (10) peut être réglé de façon coulissante suivant l'axe longitudinal du cylindre, dans une première position de réglage chaque fois la seconde face d'impact (23) pouvant être sollicitée au point mort de la course d'avance du piston et le piston pouvant être freiné par un coussin d'air comprimé au point mort de la course de retour du piston, dans une seconde position de réglage chaque fois la première face d'impact (11) pouvant être sollicitée au point mort de la course de retour du piston et le piston pouvant être freiné par un coussin d'air comprimé au point mort de la course d'avance du piston, dans une troisième position de réglage aucune des deux faces d'impact (11, 23) n'étant sollicitée et le piston pouvant être freiné aux deux points morts par chaque fois un coussin d'air comprimé.

**5.** Outil à percussion pneumatique suivant la revendication 4, caractérisé en ce que l'élément de reprise de force (10) est agencé de façon coulissante sur un élément (32) retenu élastiquement dans le couvercle (8).

**6.** Outil à percussion pneumatique suivant l'une des revendications 2 à 4, caractérisé en ce que le piston (1, 2, 3) et l'élément de reprise de force (10) sont constitués de métal et en ce que le cylindre (6, 7, 8) est formé en aluminium ou en matière synthétique renforcée par fibres.

FIG. 1

FIG. 2a

FIG. 2b

FIG. 2c

FIG. 2d

FIG. 3

FIG. 4a

FIG. 4b

FIG. 4c

FIG. 4d

FIG. 4 e

14

FIG. 5a

FIG. 5b

FIG. 5c

FIG. 5d

FIG. 6a

FIG. 6b

FIG. 6c

FIG. 6d

EP 0 452 543 B1

FIG. 7

17